(19) Europäisches Patentamt
European Patent Office
Office européen des brevets

(11) Veröffentlichungsnummer: **0 002 735**
**B1**

(12) **EUROPÄISCHE PATENTSCHRIFT**

(21) Anmeldenummer: **78101676.1**

(22) Anmeldetag: **14.12.78**

(51) Int. Cl.³: **C 07 D 317/60,**
C 07 D 405/10

# (54) Verfahren zur Herstellung von Piperonylidencrotonsäureamiden

(30) Priorität: **22.12.77 DE 2757506**

(43) Veröffentlichungstag der Anmeldung:
**11.07.79 Patentblatt 79/14**

(45) Bekanntmachung des Hinweises auf die Patenterteilung:
**10.12.80 Patentblatt 80/25**

(84) Benannte Vertragsstaaten:
**CH DE FR GB IT NL**

(56) Entgegenhaltungen:
**JOURNAL OF THE AMERICAN CHEMICAL SOCIETY, Vol. 74, November 5. '52, PETE D. GARDNER et al.: "A new approach to δ-Phenylvaleric acids", Seiten 5527-5529**

(73) Patentinhaber: **HAARMANN & REIMER GMBH**
**Postfach 138**
**D - 3450 Holzminden (DE)**

(72) Erfinder: **Schulze, Andreas, Dr.**
**Jakob-Böhme-Strasse 19**
**D - 5000 Köln 80 (DE)**
**Oediger, Hermann, Dr.**
**Roggendorfstrasse 51**
**D - 5000 Köln 80 (DE)**

(74) Vertreter: **Dill, Erwin, Dr., et al**
**c/o BAYER AG Zentralbereich Patente Marken und Lizenzen Bayerwerk**
**D - 5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 735 B1

Courier Press, Leamington Spa, England.

Verfahren zur Herstellung von Piperonylidencrotonsäureamiden

Die vorliegende Erfindung betrifft ein neues Verfahren zur Herstellung von Piperonylidencrotonsäureamiden.

Piperonylidencrotonsäureamide finden eine vielfältige technische Verwendung. So ist Piperonylidencrotonsäurepiperidid (Trivialname: Piperin) ein wichtiger Würzstoff. Er stellt das Schärfeprinzip des schwarzen Pfeffers dar (Chromatographia Band 8 (1975), Seiten 342—344). Als Würzstoffe eignen sich ferner zur Geschmacksabrundung von Pfefferzubereitungen Piperonylidencrotonsäurepyrrolidid auch Piperylin genannt (Chem. Ber. Bd. 103 (1970), Seiten 3752—3770) und Piperonylidencrotonsäureisobutylamid auch Piperlonguminin genannt (Tetrahedron Bd. 23 (1967), Seiten 1769—1781). Piperin findet ferner Verwendung als Zusatz zu germiziden Zubereitungen (US—PS 2 085 064). Piperonylidencrotonsäureamide eignen sich auch als Insektizide oder Insektizid-Synergisten (US-PS 2 487 179; Contrib. Boyce Thompson Inst. Bd. 13 (1945) Seiten 433—442; UdSSR-Pat. 222 056; DT—OS 2 413 756). Weiterhin eignet sich Piperin auch als Analeptikum bei Morphin- oder Barbituratvergiftungen (J. Res. Indian Med. Bd. 8 (1973), Seiten 1—9 und Bd. 9 (1974) Seiten 17—22).

Es sind deshalb bereits verschiedene Verfahren zur Herstellung von Piperonylidencrotonsäureamiden insbesondere von Piperin vorgeschlagen worden. So wird nach einem Herstellungsverfahren zunächst Piperonal in einem dreistufigen Verfahren in Piperonylidenacetaldehyd überführt, dieser mit Malonsäurehalbester zum Piperonylidencrotonsäureester kondensiert und dieser über weitere drei Stufen mittels Piperidin in das entsprechende Piperidid überführt (Chem. Ber. Bd. 108 (1975), Seiten 95—108).

Dieses Verfahren ist jedoch wegen seiner vielen Verfahrensschritte und der unbefriedigenden Ausbeuten — die Gesamtausbeute beträgt lediglich 41% — unwirtschaftlich und kommt deshalb für eine Darstellung von Piperin in technischem Maßstab nicht in Betracht.

Weiterhin hat man Piperin durch Kondensation von Piperonylidenacetaldehyd mit Piperidinocarbonylmethyl-triphenylphosphoniumjodid hergestellt (Pharm. Chem. J. Bd. (1971), Seiten 462 bis 464). Diese Herstellung hat jedoch den Nachteil, daß die als Reaktionskomponenten einzusetzenden Verbindungen ihrerseits erst durch mehrstufige Verfahren hergestellt werden müssen und daß bei der Kondensation große Mengen Triphenylphosphinoxid entstehen, die sich nur umständlich vom Piperin abtrennen lassen. Auch dieses Verfahren ist daher für eine Piperin-Synthese in technischem Maßstab ungeeignet.

Es wurde nun gefunden, daß man bekannte und neue Piperonylidencrotonsäureamide der Formel

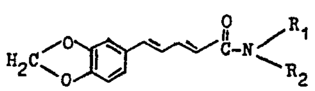

I

in der

$R_1$ und $R_2$ unabhängig voneinander für Wasserstoff, oder einen gegebenenfalls substituierten aliphatischen, araliphatischen oder aromatischen Kohlenwasserstoffrest stehen oder gemeinsam mit dem Stickstoffatom einen heterocyclischen Ring bilden, mit der Maßgabe, daß $R_1$ und $R_2$ nicht gleichzeitig Wasserstoff bedeuten,

auf einfache Weise in ausgezeichneter Ausbeute herstellen kann, wenn man Piperonal mit Crotonsäureamiden der Formel

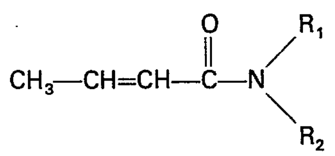

II

in der

$R_1$ und $R_2$ die vorstehend angegebene Bedeutung haben,

in Gegenwart von Alkalihydroxiden und unter den Reaktionsbedingungen inerten dipolaren aprotischen Verdünnungsmitteln umsetzt.

Das erfindungsgemäße Verfahren zeichnet sich gegenüber den aus dem Stand der Technik bekannten Verfahren durch eine wesentlich vereinfachte Arbeitsweise (wenige Reaktionsstufen, Verwendung technisch ohne besondere Vorsichtsmaßnahmen handhabbare Kondensationsmittel) und durch erheblich verbasserte Ausbeuten aus. Nach dem erfindungsgemäßen Verfahren lassen sich Piperonylidencrotonsäureamide ohne Schwierigkeiten in technischem Maßstab herstellen. Die Verbindungen fallen in hoher Reinheit an.

Es war zwar bereits bekannt, Aldehyde mit 3-Methylbutencarbonsäureestern, 3-Methylbutencarbonsäureamiden oder Crotonsäureestern zu kondensieren (US—PS 2 951 853; Chem. Ber. Bd. 106 (1973), Seiten 2643—2647). Die Kondensationen wurden jedoch unter Verwendung mindestens

äquimolarer Mengen starker Kondensationsmittel wie Alkalimetallen, Alkaliamiden, Alkalihydriden, oder metallorganischen Verbindungen wie Phenylnatrium oder Triphenylmethylkalium, bezogen auf die Menge Alkensäureester une völligem Ausschluß von Wasser durchgeführt.

Es war daher überraschend, daß die erfindungsgemäße Umsetzung bereits mit katalytischen Mengen als schwächer wirksam bekannter Kondensationsmittel, wie Natrium- oder Kaliumhydroxid, auch ohne Ausschluß von Wasser in hohen Ausbeuten verläuft. Im Hinblick darauf, daß Piperonal in seiner Reaktionsfähigkeit mit 4-Methoxybenzaldehyd vergleichbar ist, und da bekannt war, daß desaktivierte Aldehyde wie 4-Methoxybenzaldehyd mit Crotonsäureäthylester selbst in Gegenwart starker Kondensationsmittel wie Natriumamid nur in 12%iger Ausbeute zum gewünschten Kondensationsprodukt reagieren, (Chem. Ber. Bd. 106 (1973), Seiten 2643—2647) war zu erwarten, daß unter den erfindungsgemäßen Reaktionsbedingungen keinerlei Umsatz stattfinden würde.

Es war ferner bekannt, daß sich Piperonal mit Äthylidenmalonsäureester mit Kaliumhydroxid in Äthanol kondensieren läßt (J. Am. Chem. Soc. Bd. 74 (1952), Seiten 5527—5529). Ersetzt man in dieser Reaktion jedoch den als sehr reaktionsfähig bekannten Äthylidenmalonsäureester durch die wesentlich reaktionsträgeren Crotonsäureamide, so fallen die Ausbeuten an Kondensationsprodukt derartig stark ab, (auf etwa 10% der Theorie), sodaß diese Reaktion für die Herstellung von Piperonylidencrotonsäureamiden uninteressant ist.

Wesentlich für den überraschend, günstigen Verlauf der erfindungsgemäßen Kondensationsreaktion von Piperonal mit Crotonsäureamiden ist, daß diese Kondensation nicht wie in den vorgenannten Kondensationsreaktionen in unpolaren oder polaren protischen organischen Verdünnungsmitteln, sondern in dipolaren aprotischen organischen Verdünnungsmitteln vorgenommen wird.

Als unter den Reaktionsbedingungen inerte, dipolare aprotische organische Verdünnungsmittel seien beispielsweise genannt:

Säureamide, z.B. Dialkylformamide oder Dialkylacetamide wie Dimethylformamid oder Dimethylacetamid, 1-Methyl-2-pyrrolidinon; Tetraalkylharnstoffe wie Tetramethylharnstoff; Sulfoxide, z.B. Dialkylsulfoxide wie Dimethylsulfoxid und Tetrahydrothiophen-1-oxid; Sulfone wie Tetrahydrothiophen-1,1-dioxid; oder Hexaalkylphosphorsäuretriamide wie Hexamethylphosphorsäuretriamid; oder 1-Methyl-1-oxo-phospholin. Besonders bewährt hat sich Dimethylsulfoxid.

Die Mengen, in denen die dipolaren aprotischen organischen Verdünnungsmittel eingesetzt werden, können in weiten Grenzen schwanken; im allgemeinen hat es sich bewährt, je Mol Piperonal 50—500, vorzugsweise 100—350 ml Verdünnungsmittel einzusetzen. Eine Erhöhung der Verdünnungsmittelmenge über die angegebene Höchstmenge von 500 ml hinaus ist möglich, bringt aber keinen Vorteil.

Von den im erfindungsgemäßen Verfahren als Kondensationsmittel verwendbaren Alkalihydroxiden, wie Lithium-, Natrium-, und Kaliumhydroxid, werden vor allem Natrium- und Kaliumhydroxid eingesetzt. Kaliumhydroxid hat sich besonders bewährt.

Die Alkalihydroxide können sowohl als wasserfreie Verbindungen wie auch in Form wäßriger, z.B. 40 bis 50%iger wäßriger Lösungen, eingesetzt werden.

Die Alkalihydroxide werden im allgemeinen in Mengen von 0,05—0,5 Mol, vorzugsweise 0,1—0,2 Mol je Mol Piperonal angewendet.

Die erfindungsgemäße Kondensation wird im allgemeinen bei Temperaturen zwischen —10°C und +70°C, vorzugsweise zwischen +10°C und +30°C vorgenommen. Bei Verwendung sehr geringer Mengen an Verdünnungsmittel und Alkalihydroxid können Reaktionstemperaturen von 65—70°C vorteilhaft sein.

Die Reaktion kann bei Normaldruck oder erhöhtem Druck durchgeführt werden.. Im allgemeinen arbeitet man bei Normaldruck. Vorzugsweise arbeitet man in einer Inertgasatmosphäre, beispielsweise unter Stickstoff oder Argon.

In dem erfindungsgemäßen Verfahren setzt man zweckmäßigerweise die beiden Reaktionspartner in etwa äquimolaren Mengen ein; es hat sich bewährt, je Mol Piperonal 1 bis 1,2 Mol Crotonsäureamid, vorzugsweise 1 bis 1,1 Mol Crotonsäureamid zu verwenden.

Die Piperonylidencrotonsäureamide fallen in dem erfindungsgemäßen Verfahren im allgemeinen im Laufe der Reaktion als feste Niederschläge aus. Diese können durch Umkristallisieren gereinigt werden. Das nach dem Abfiltrieren des Piperonylidencrotonsäureamid-Niederschlags anfallende Verdünnungsmittel kann ohne Reinigung erneut verwendet werden.

Der Verlauf des erfindungsgemäßen Verfahrens sei an folgendem Reaktionsschema erläutert:

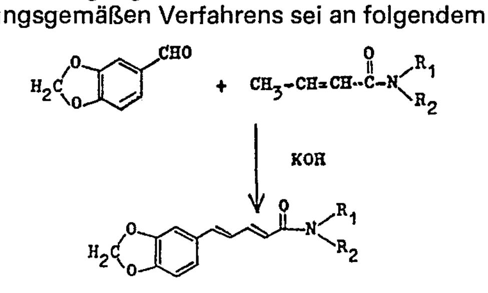

Für $R_1$ und $R_2$ seien beispielsweise genannt:

Als gegebenenfalls substituierte aliphatische Kohlenwasserstoffreste vor allem $C_1$—$C_6$-Alkyl-, $C_1$—$C_6$-Alkenyl- und 5- und 6-gliedrige Cycloalkylreste. Beispielsweise der Methyl-, Äthyl-, n-Propyl-, i-Propyl-, n-Butyl-, sec.-Butyl-, i-Pentyl-, n-Hexyl-, i-Hexyl-, Allyl-, Cyclopentyl- und Cyclohexylrest, ferner durch $C_1$—$C_4$-Alkylgruppen substituierte Cyclopentyl und Cyclohexylreste wie der 4-Methyl- und 2,4-Dimethyl-cyclohexylrest. Als Substituenten für die Alkylreste kommen vor allem Halogenatome wie das Chloratom und die Hydroxy Gruppe in Betracht. Beispiele für substituierte Alkylreste: der 2-Chloräthyl- und 2-Hydroxyäthyl-Rest.

Als gegebenenfalls substituierte araliphatische und aromatische Kohlenwasserstoffreste, vor allem der Benzyl- und Phenylrest und durch Halogenatome, z.B. Chlor- oder Bromatome, durch $C_1$—$C_4$-Alkyl- und $C_1$—$C_4$-Alkoxygruppen substituierte Benzyl- und Phenylreste wie der 4-Methyl- und 3-Chlorbenzyl-Rest und der 3-Chlor-, 2,4-Dichlor-, 2-Brom-4-methyl-, 4-Äthyl- und der 4-Methoxyphenyl-Rest.

Als Heterocyclen, die $R_1$ und $R_2$ zusammen mit dem Amidstickstoff bilden können vor allem 5- bis 7-gliedrige, gegebenenfalls weitere Heteroatome wie Sauerstoff, Schwefel oder Stickstoff enthaltende Heterocyclen wie der Piperidin-, Pyrrolidin-, Morpholin- und Hexamethylenimin-Ring. Diese Heterocyclen können auch substituiert sein, z.B. durch $C_1$—$C_4$-Alkylgruppen: z.B. 2-, 3- und 4-Methylpiperidin, 2,3-, 2,4- und 2,6-Dimethylpiperidin, 2-Äthyl-piperidin, 2,4,6-Trimethylpiperidin.

Die erfindungsgemäß als Ausgangsverbindungen zu verwendenden Crotonsäureamide sind bekannt oder können nach an sich bekannten Verfahren hergestellt werden, beispielsweise aus Crotonsäurechlorid und den entsprechenden Aminen (Helv. Chim. Acta Bd. 38 (1955), Seiten 1085—1095) oder aus den entsprechenden Crotonsäureammoniumsalzen entweder durch Abspaltung von Wasser bei erhöhter Temperatur, gegebenenfalls in Gegenwart saurer Katalysatoren oder durch Umsetzung mit anorganischen Säurehalogeniden, z.B. Thionylchlorid (Houben-Weyl, Methoden der organischen Chemie, Bd. XI/2, Seiten 3—9, Thieme Verlag Stuttgart 1958).

Als Vertreter der erfindungsgemäß umzusetzenden Crotonsäureamide seinen beispielsweise genannt:

Crotonsäurepiperidid,
Crotonsäurepyrrolidid,
Crotonsäuremorpholid,
Crotonsäurehexamethylenimid,
Crotonsäure-(2-methylpiperidid),
Crotonsäure-(3-methylpiperidid),
Crotonsäure-(4-methylpiperidid),
Crotonsäure-(2-pentylpiperidid),
Crotonsäure-(4-pentylpiperidid),
Crotonsäure-(2,4,6-trimethylpiperidid),
Crotonsäure-(2,6-dimethylpiperidid),
Crotonsäure-(2,4-dimethylpiperidid),
Crotonsäure-(2-äthylpiperidid),
Crotonsäure-(2,3-dimethylpiperidid),
Crotonsäuremethylamid,
Crotonsäureäthylamid,
Crotonsäurepropylamid,
Crotonsäureallylamid,
Crotonsäurebutylamid,
Crotonsäureisobutylamid,
Crotonsäure-isopentylamid,
Crotonsäure-cyclohexylamid,
Crotonsäure-(3-äthyl-heptylamid),
Crotonsäurebenzylamid,
Crotonsäure-(3,4-methylendioxy-anilid),
Crotonsäure-anilid,
Crotonsäure-(2-brom-4-methyl-anilid),
Crotonsäure-dimethylamid,
Crotonsäure-diäthylamid,
Crotonsäure-dipropylamid,
Crotonsäure-diisopropylamid,
Crotonsäure-diallylamid,
Crotonsäure-dibutylamid,
Crotonsäure-diisobutylamid,
Crotonsäure-dicyclohexylamid,
Crotonsäure-(di-β-chloräthyl-amid),
Crotonsäure-(di-β-hydroxyäthyl-amid).

Als Vertreter der nach dem erfindungsgemäßen Verfahren herstellbaren Piperonylidencrotonsäureamide seien beispielsweise genannt:

Piperonylidencrotonsäurepiperidid,
Piperonylidencrotonsäurepyrrolidid,
Piperonylidencrotonsäuremorpholid,
Piperonylidencrotonsäurehexamethylenimid,
Piperonylidencrotonsäure-(2-methylpiperidid),
Piperonylidencrotonsäure-(3-methylpiperidid),
Piperonylidencrotonsäure-(4-methylpiperidid),
Piperonylidencrotonsäure-(2-pentylpiperidid),
Piperonylidencrotonsäure-(4-pentylpiperidid),
Piperonylidencrotonsäure-(2,4,6-trimethylpiperidid),
Piperonylidencrotonsäure-(2,6-dimethylpiperidid),
Piperonylidencrotonsäure-(2,4-dimethylpiperidid),
Piperonylidencrotonsäure-(2-äthylpiperidid),
Piperonylidencrotonsäure-(2,3-dimethylpiperidid),
Piperonylidencrotonsäuremethylamid,
Piperonylidencrotonsäureäthylamid,
Piperonylidencrotonsäurepropylamid,
Piperonylidencrotonsäureallylamid,
Piperonylidencrotonsäurebutylamid,
Piperonylidencrotonsäureisobutylamid,
Piperonylidencrotonsäure-isopentylamid,
Piperonylidencrotonsäure-cyclohexylamid,
Piperonylidencrotonsäure-(3-äthyl-heptylamid),
Piperonylidencrotonsäurebenzylamid,
Piperonylidencrotonsäure-(3,4-methylendioxy-anilid),
Piperonylidencrotonsäure-anilid,
Piperonylidencrotonsäure-(2-brom-4-methyl-anilid),
Piperonylidencrotonsäure-dimethylamid,
Piperonylidencrotonsäure-diäthylamid,
Piperonylidencrotonsäure-dipropylamid,
Piperonylidencrotonsäure-diisopropylamid,
Piperonylidencrotonsäure-diallylamid,
Piperonylidencrotonsäure-dibutylamid,
Piperonylidencrotonsäure-diisobutylamid,
Piperonylidencrotonsäure-dicyclohexylamid,
Piperonylidencrotonsäure-(di-β-chloräthyl-amid),
Piperonylidencrotonsäure-(di-β-hydroxyäthyl-amid).

Bei den in den folgenden Beispielen angegebenen Teilen handelt es sich um Gewichtsteile, falls nichts anderes angegeben wurde.

Beispiel 1

Die Lösung von 30 Teilen (0,2 Mol) Piperonal, 32 Teilen (0,209 Mol) Crotonsäurepiperidid in 50 Volumenteilen Dimethylsulfoxid wird unter Stickstoff bei 20 bis 25°C mit 2,2 Teilen (0,04 Mol) gepulvertem Kaliumhydroxid versetzt. Die Reaktionsmischung wird 4 Stunden bei 25 bis 30°C und anschließend 10 Stunden bei 25°C gerührt. Der Niederschlag wird abgesaugt, mit Wasser gewaschen und getrocknet.

Ausbeute: 45,6 Teile rohes Piperonylidencrotonsäurepiperidid.

Nach dem Umkristallisieren aus Äthylacetate beträgt die Ausbeute an reinem Piperonylidencrotonsäurepiperidid 42 Teile (= 73% der Theorie). Schmelzpunkt: 129—130°C.

Piperonylidencrotonsäurepiperidid wurde in vergleichbarer Ausbeute und Reinheit erhalten, wenn statt der 50 Volumenteile Dimethylsulfoxid 50 Volumenteile Tetrahydrothiophen-1,1-dioxid, N-Methylpyrrolidinon oder Dimethylformamid als Lösungsmittel verwendet wurde.

Beispiele 2 bis 8

1 Mol Piperonal wird mit 1,05 Mol eines in der nachstehenden Tabelle angegebenen Crotonsäureamides in Gegenwart von 0,2 Mol Kaliumhydroxid unter den in Beispiel 1 angegebenen Bedingungen umgesetzt. Es werden die in der Tabelle aufgeführten Piperonylidencrotonsäureamide in den ebenfalls in der Tabelle angegebenen Ausbeuten erhalten.

Tabelle

Formeln der eingesetzten Crotonsäureamide (a) und der aus ihnen erhaltenen Piperonyliden-crotonsäureamide (b)

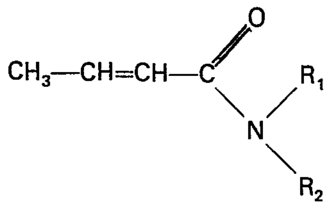

(a)

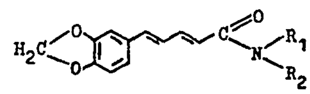

(b)

| Beispiel Nr | 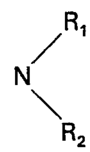 | Ausbeute in % der Theorie | Fp in °C |
|---|---|---|---|
| 3 | 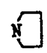 | 68 | 142—143 |
| 4 | 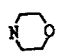 | 70 | 167—168 |
| 5 | $HN—CH_2—CH(CH_3)_2$ | 71 | 165—166 |
| 6 | $N(C_2H_5)_2$ | 69 | 94—95 |
| 7 | $N(CH_2—CH{=}CH_2)_2$ | 70 | 75—76 |
| 8 | 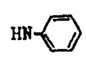 | 72 | 197—198 |

Beispiel 9

In eine Lösung von 600 Teilen Piperonal, 639 Teilen Crotonsäurepiperidid in 1000 Volumenteilen Dimethylsulfoxid läßt man bei 25°C unter Stickstoff 88 Teile 50%ige Kalilauge unter kräftigem Rühren schnell einfließen. Man hält die Temperatur der Reaktionsmischung durch Kühlen auf 28—30°C und rührt nach dem Auskristallisieren des Reaktionsgemisches noch einige Zeit bei Raumtemperatur nach. Das Kristallisat wird abgesaugt, mit Wasser gewaschen und getrocknet.

Man erhält 911 Teile Roh-Piperonylidencrotonsäurepiperidid. Es wird durch Umkristallisieren aus Äthylacetat gereinigt. Ausbeute: 870 Teile reines Piperonylidencrotonsäurepiperidid (= 76% der Theorie), Schmelzpunkt 129—130°C.

Beispiel 10

Die Lösung von 15,0 Teilen (0,1 Mol) Piperonal und 17,0 Teilen (0,11 Mol) Crotonsäurepiperidid in 10 Volumenteilen Dimethylsulfoxid wird bei 25°C mit 0,8 ml 50%-iger wässriger KOH versetzt. Anschließend wird die Reaktionsmischung 8 Stunden bei 60—65°C gerührt. Nach dem Abdestillieren des Lösungsmittels im Vakuum versetzt man das Reaktionsgemisch mit 40 ml Wasser. Der Niederschlag wird abgesaugt, mit Wasser gewaschen und getrocknet.

Nach dem Umkristallisieren aus Äthylacetat werden 22 Teile (=77% der Theorie) reines Piperonylidencrotonsäurepiperidid erhalten Schmelzpunkt 129—130°C.

Beispiel 11

Die Lösung von 15 Teilen (0,1 Mol) Piperonal und 17 Teilen (0,11 Mol) Crotonsäurepiperidid in 50 Volumenteilen Dimethylsulfoxid wird bei 26°C unter Stickstoff mit 1,65 Teilen 50%-iger wässriger Natronlauge versetzt. Die Reaktionsmischung wird 8 Stunden bei 26 bis 30°C gerührt, anschließend das angefallene Kristallisat abgesaugt, mit Wasser gewaschen und getrocknet.

Ausbeute an Roh-Piperonylidencrotonsäurepiperidid: 20 Teile.

Nach dem Umkristallisieren aus Äthylacetat beträgt die Ausbeute an reinem Piperonylidencrotonsäurepiperidid 17 Teile (= 60% der Theorie). Schmelzpunkt 129—130°C.

**Patentansprüche**

1. Verfahren zur Herstellung von Piperonylidencrotonsäureamiden der Formel

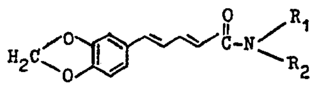

I

in der

$R_1$ und $R_2$ unabhängig voneinander für Wasserstoff, oder einen gegebenenfalls substituierten aliphatischen, araliphatischen oder aromatischen Kohlenwasserstoffrest stehen oder gemeinsam mit dem Stickstoffatom, einen heterocyclischen Ring bilden, mit der Maßgabe, daß $R_1$ und $R_2$ nicht gleichzeitig Wasserstoff bedeuten,
dadurch gekennzeichnet, daß man Piperonal mit Crotonsäureamiden der Formel

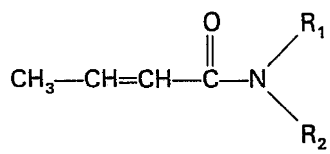

II

in der

$R_1$ und $R_2$ die vorstehend angegebene Bedeutung haben,
in Gegenwart von Alkalihydroxiden und unter den Reaktionsbedingungen inerten dipolaren aprotischen Verdünnungsmitteln umsetzt.

2. Verfahren gemäß Anspruch 1, dadurch gekennzeichnet, daß man die Alkalihydroxide in einer Menge von 0,05—0,5 Mol je Mol Piperonal einsetzt.

3. Verfahren gemäß Anspruch 1 und 2, dadurch gekennzeichnet, daß man als inerte dipolare aprotische Verdünnungsmittel Dialkylformamide, Dialkylacetamide, 1-Methyl-2-pyrrolidinon, Tetraalkylharnstoffe, Dialkylsulfoxide, Sulfone, Hexaalkylphosphorsäuretriamide oder 1-Methyl-1-oxo-phospholin verwendet.

4. Verfahren gemäß Anspruch 1 bis 3, dadurch gekennzeichnet, daß man als inertes dipolares aprotisches Verdünnungsmittel Dimethylsulfoxid verwendet.

**Revendications**

1. Procédé de production d'amides d'acide pipéronylidène-crotonique de formule:

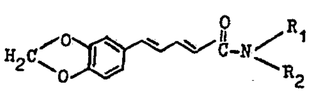

I

(dans laquelle $R_1$ et $R_2$ représentent, indépendamment l'un de l'autre, de l'hydrogène ou un reste hydrocarboné aliphatique, araliphatique ou aromatique éventuellement substitué ou forment un noyau hétérocyclique en commun avec l'atome d'azote, à condition que $R_1$ et $R_2$ no représentent pas simultanément de l'hydrogène), caractérisé en ce qu'il consiste à faire réagir du pipéronal avec des amides d'acide crotonique de formule:

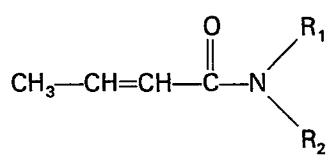

II

(dans laquelle $R_1$ et $R_2$ ont les définitions données ci-dessus) en présence d'hydroxydes de métaux alcalins et de diluants aprotiques dipolaires inertes dans les conditions réactionnelles.

2. Procédé suivant la revendication 1, caractérisé en ce que les hydroxydes de métaux alcalins sont utilisés en une quantité di 0,05 à 0,5 mole par mole de pipéronal.

3. Procédé suivant l'une des revendications 1 et 2, caractérisé en ce qu'il consiste à utiliser comme diluants aprotiques dipolaires inertes des dialkylformamides, des dialkylacétamides, la 1-méthyl-2-pyrrolidinone, des tétra-alkylurées, de sulfoxydes dialkyliques, des sulfones, des triamides d'acides hexa-alkylphosphoriques ou la 1-méthyl-1-oxophospholine.

4. Procédé suivant l'une quelconque des revendications 1 à 3, caractérisé en ce qu'il consiste à utiliser le diméthylsulfoxyde comme diluant aprotique dipolaire inerte.

**Claims**

1. Process for the preparation of piperonylidene-crotonic acid amides of the formula

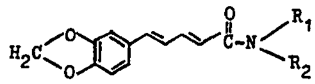 I

in which

$R_1$ and $R_2$ independently of one another represent hydrogen or an optionally substituted aliphatic, araliphatic or aromatic hydrocarbon radical, or, together with the nitrogen atom, form a heterocyclic ring, with the proviso that $R_1$ and $R_2$ do not simultaneously denote hydrogen, characterised in that piperonal is reacted with crotonic acid amides of the formula:

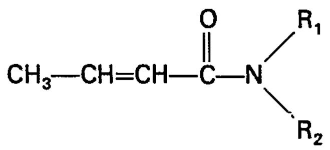 II

in which

$R_1$ and $R_2$ have the meaning indicated above, in the presence of alkali metal hydroxides and dipolar aprotic diluents which are inert under the reaction conditions.

2. Process according to Claim 1, characterised in that the alkali metal hydroxides are employed in an amount of 0.05—0.5 mol per mol of piperonal.

3. Process according to Claims 1 and 2, characterised in that dialkylformamides, dialkylacetamides, 1-methyl-2-pyrrolidinone, tetraalkylureas, dialkylsulphoxides, sulphones, hexaalkylphosphoric acid triamides or 1-methyl-1-oxo-phospholine are used as the inert dipolar aprotic diluents.

4. Process according to Claims 1 to 3, characterised in that dimethylsulphoxide is used as the inert dipolar aprotic diluents.